# EUROPEAN PATENT APPLICATION

(11) **EP 4 404 211 A1**
(43) Date of publication of application: **24.07.2024**
(21) Application number: 21957337.5
(22) Date of filing: 07.12.2021
(51) Int. Cl.: G16H 40/67

(54) **INTELLIGENT VACCINE NEBULIZATION SYSTEM, AND USAGE METHOD**

(30) Priority: 14.09.2021 CN 202111071268
(71) Applicant: Qingdao Future Medical Technology Co., Ltd., Qingdao, Shandong 266102 (CN)
(72) Inventor: WANG, Qixu, Qingdao, Shandong 266102 (CN); SUN, Zhongjin, Qingdao, Shandong 266102 (CN); WANG, Weilai, Qingdao, Shandong 266102 (CN)
(74) Representative: Cabinet Beau de Loménie
(86) International application number: PCT/CN2021/135893
(87) International publication number: WO 2023/040073

(57) **Abstract**

An intelligent vaccine nebulization system, and a usage method, which are characterized in that the intelligent vaccine nebulization system comprises an intelligent vaccine nebulization apparatus, a mist storage tank and a cloud server, wherein the intelligent vaccine nebulization apparatus comprises a host case body, and an intelligent main control module and functional modules on the host case body. The functional modules comprise an identity information input module, a vaccine information input module, a vaccine temporary storage module, a quantitative pipetting module, an aerosol output module, a mist storage tank management module, a human-computer interaction module and a communication interface. The intelligent main control module is connected to the functional modules, and exchanges information with the cloud server, so as to guarantee strict and accurate vaccination and management. Control is performed by the intelligent main control module, and by means of the steps of identity information and vaccine information input, vaccine temporary storage, quantitative pipetting, aerosol output, mist storage tank management, etc., access to the cloud server for vaccine recipients and nebulization-related information records thereof is achieved, such that automatic, efficient and convenient vaccine nebulization and vaccination can be achieved..

## Description

### TECHNICAL FIELD

The present disclosure belongs to the technical field of intelligent system for vaccination, and also relates to the technical field of medical information processing and the technical field of communication, and is specifically to an intelligent vaccine nebulization system and a method of use.

### BACKGROUND

A considerable amount of research in the field of vaccines has shown that for infectious diseases that invade the body through the respiratory mucosa, local vaccination is more effective and safer than peripheral vaccination. Local administration of vaccines, such as the aerosolized inhaled COVID-19 vaccine, initiates the defense against viruses at the mucosal level, and can prevent blood-borne infections caused by needle contamination during injection.

The effectiveness of the aerosolized inhaled vaccine lies in its ability to activate the body's mucosal immunity. Mucosal immunity is associated with Immunoglobulin A (IgA), which newborns acquire from breast milk and begin producing as they grow older. IgA is distributed in breast milk, saliva, and mucosal secretions in the gastrointestinal tract, respiratory tract, and genitourinary tract, serving as the first line of defense against pathogen invasion.

The aerosolized inhaled COVID-19 vaccine can stimulate the production of IgA: the inhaled vaccine transports the antigen to the nasopharyngeal-associated lymphoid tissue (NALT) and presents it to antigen-presenting cells (APCs), which then present it to T cells, leading to the activation of IgA+B cell development. Antigen-specific CD4+ cells and IgA+B cells migrate to the upper respiratory tract via the thoracic duct and blood circulation. Under the influence of cytokines secreted by CD4+ cells, IgA+B cells differentiate into IgA+ plasma cells, producing dimeric forms of IgA. This IgA then binds to polymeric Ig receptors on epithelial mucosal cells, becoming secretory IgA, which is eventually released onto the surface of the upper respiratory tract for protective purposes.

At the Pujiang Innovation Forum on June 3, 2021, Chen Wei, a researcher at the Academy of Military Sciences and a member of the Chinese Academy of Engineering, announced that her team's adenovirus vector-based aerosolized inhaled COVID-19 vaccine is undergoing the process of emergency use approval. This implies that future COVID-19 vaccinations may be administered without injections, requiring only inhalation of the aerosolized vaccine and a brief breath-holding period.

In comparison to injectable vaccines, the advantages of the aerosolized inhaled COVID-19 vaccine are evident, including:
1. Reduced dosage requirements, approximately one-fifth that of injectable vaccines. The absence of vials resolves manufacturing bottlenecks and lessens the burden on vaccine logistics and transportation.
2. Increased production capacity, as one-fifth of the traditional injection vaccine dosage can now serve five individuals, leading to a significant boost in overall production.
3. Enhanced immune response, as the aerosolized vaccine triggers a triple immune response: humoral, cellular, and mucosal immunity, offering superior protection compared to the two types of immunity generated by injectable vaccines: humoral and cellular.
4. Rapid immune activation, with the aerosolized vaccine quickly taking effect in the nasal passages, throat, trachea, alveoli, and mucous membranes upon inhalation.
5. Enhanced vaccine accessibility, as the painless nature of the inhaled vaccine makes it a preferred option for individuals with needle-related fears or phobias, particularly among children during subsequent vaccination campaigns.

### SUMMARY

### Technical Problem

However, the conventional process for administering the aerosolized inhaled COVID-19 vaccine involves a nurse extracting an appropriate vaccine volume from a container using a pipette and placing it into the nebulizer. Subsequently, the nurse or the vaccine recipient activates the nebulizer. This manual process is inefficient and time-consuming, resulting in low turnover rates for the nebulizer and a higher likelihood of errors. Consequently, this method fails to meet the demands of large-scale, time-sensitive, and high-efficiency vaccination campaigns involving the aerosolized inhaled COVID-19 vaccine.

As a result, the urgent need to develop an intelligent vaccine nebulization system and a user-friendly approach to enable automated, efficient, and convenient administration of the aerosolized inhaled COVID-19 vaccine has become a crucial aspect in combating the COVID-19 pandemic and remains a pressing issue within the realm of technical development.

### SOLUTION TO THE PROBLEM

### Technical Solution

In order to solve the above-mentioned problems in the prior art, the present invention provides an intelligent vaccine nebulization system and a method of use, which can realize automatic, efficient and convenient nebulization vaccination.

The purpose of the present invention is achieved through the following technical solutions.

An intelligent vaccine nebulization system comprising: an intelligent vaccine nebulization device, a mist storage tank and a cloud server, wherein the intelligent vaccine nebulization device includes a main cabinet, an intelligent main control module and a plurality of functional modules provided on the main cabinet, wherein the functional modules include an identity information input module, a vaccine information input module, a temporary vaccine storage module, a dosing transfer module, an aerosol output module, a mist storage tank management module, a human-computer interaction module and a communication interface, in which the intelligent main control module is respectively connected with the functional modules and the intelligent main control module is communicated with the cloud server;
the identity information input module is configured to input ID information of the vaccine recipient and send it the cloud server through the intelligent main control module;
the vaccine information input module is configured to identify vaccine manufacturers, vaccine types and production batch numbers and supports user-defined or automatically downloads of storage parameters, nebulization parameters and usage parameters from the cloud server based on the vaccine types;
the temporary vaccine storage module is configured to accommodate vaccine containers from different vaccine manufacturers, ensuring proper storage conditions to maintain the vaccine's effectiveness throughout the vaccination process;
the dosing transfer module is configured to automatically and accurately extract specified vaccine dosage according to different nebulized vaccination requirements and to transfer the measured amount of vaccine to the aerosol output module;
the aerosol output module is configured to automatically complete the nebulization based on the specific nebulization parameters for each vaccine, which is provided with an aerosol outlet through which the aerosol is filled into the mist storage tank;
the mist storage tank management module is configured to perform detection and to install the mist storage tank before nebulization, to distribute the mist storage tank, in which vaccine after nebulization by the aerosol output module is filled into, to the vaccine recipient for inhalation, in which the installation of the mist storage tank is to connect the mist storage tank to the aerosol outlet of the aerosol output module and the detection before nebulization is to check whether the mist storage tank is inserted into the aerosol outlet and is in place;
the human-computer interaction module is configured to display vaccination information and a human-machine interface, providing prompts to the management personnel and the vaccine recipients;
the communication interface is configured to connect to communicable devices;
the intelligent main control module is configured to control the functional modules of the intelligent vaccine nebulization system, to access the information of the vaccine recipient and other nebulization-related information on the cloud server, and to complete the control of a whole process including vaccine storage, nebulization and mist storage tank output based on the vaccine recipient and vaccine information;
the mist storage tank is a container for storing vaccine aerosol, which is connected to the aerosol output module to receive the vaccine aerosol produced by the aerosol output module that is inhaled by the vaccine recipient for vaccination;
the cloud server is configured to interact with the intelligent main control module to access vaccination information, vaccine information and the vaccine recipient information; and
the main cabinet is configured to accommodate the functional modules and used to receive and store the vaccines and the mist storage tank and to output the mist storage tank.

As an improvement, the intelligent vaccine nebulization system further includes an inhalation quality detection module configured to verify if a vaccination process is normally run according to predetermined vaccination quality standards; if not, to generate notifications for manual intervention or to initiate preset operations automatically.

As an improvement, the inhalation quality detection module includes a detection camera device or/and a working process detection sensor device; wherein the working process detection sensor device includes an installation detection sensor, a photoelectric sensor, a body posture sensor and an air pressure sensor, which is configured to determine whether the inhalation posture and the volume inhaled by the vaccine recipient meet the preset vaccination requirements by providing feedbacks through the human-machine interface on the human-computer interaction module and to determine whether a vaccination process is finished on the basis of inhalation detected by providing feedbacks on the human-machine interface on the human-computer interaction module; and the vaccine recipient is instructed by the human-machine interface of the human-computer interaction module to put the working process detection sensor device back in place.

As an improvement, the intelligent vaccine nebulization system further includes a disposable mist storage tank recovery device configured to recover and treat medical wastes, and the vaccine recipient is instructed by the human-machine interface of the human-computer interaction module to put the used mist storage tank to designated recycling place.

As an improvement, the disposable mist storage tank recovery device includes a waste recovery processing chamber, a mist storage tank recovery opening and a mist storage tank recovery chamber door; the mist storage tank management module includes a mist storage tank management chamber and a mist storage tank management chamber door; the aerosol output module includes a mist generation device, a mist storage tank output device and a mist storage tank extraction outlet; and the vaccine temporary storage module includes a vaccine storage chamber, a vaccine storage chamber door, a vaccine temporary storage rack and a controllable low-temperature refrigeration device configured to maintain vaccines in a low-temperature environment; the controllable low-temperature refrigeration device is further configured to maintain the temperature of the dosing transfer module and the aerosol output module.

As an improvement, the main cabinet includes a shell, wherein the communication interface, the human-computer interaction module, the mist storage tank management chamber door, the mist storage tank extraction outlet, the mist storage tank recovery opening, the vaccine storage chamber door and the identity information input module are disposed on the shell.

As an improvement, the human-computer interaction module includes a display module and an audio guide module; the display module is configured to display vaccination information and the human-machine interface, and the audio guide module is configured to generate guiding or warning sounds to management personnel or the vaccine recipient; the camera device includes a detection camera and a detection camera display and the detection camera and the detection camera display are provided on a top part of the shell of the main cabinet.

As an improvement, the dosing transfer module includes a vaccine information scanner, a pipetting needle, a programmable dosing pump and a three-dimensional moving device, wherein the vaccine information scanner is arranged in the vaccine storage chamber, the pipetting needle is connected to the programmable dosing pump and the programmable dosing pump is provided on the three-dimensional moving device; the programmable dosing pump and the three-dimensional moving device are controlled by the intelligent main control module.

As an improvement, the mist storage tank includes a one-way valve, a tank body and a cover; wherein the one-way valve serves as the connection point between the mist storage tank and the aerosol output module; when the mist storage tank is disconnected from the aerosol output module, the one-way valve ensures that the aerosol locked in the mist storage tank and does not escape; when the user opens the cover to inhale the aerosol in the mist storage tank, the air flows into the mist storage tank through the one-way valve, which is convenient for the vaccine recipient to inhale the aerosol in the mist storage tank.

A method of using the intelligent vaccine nebulization system includes:
Step 1: reading the vaccine recipient's ID information, connecting to the cloud server, retrieving personal information, vaccine type and vaccination requirements to determine whether the vaccination is permitted;
Step 2: controlling the dosing transfer module to automatically and accurately extract required vaccine dosage according to dosage requirement of vaccine nebulization and depositing it into the aerosol output module;
Step 3: controlling the mist storage tank management module to determine whether the mist storage tank is installed in place; if usage requirements are not met, the mist storage tank is automatically installed or replaced;
Step 4: controlling the aerosol output module to automatically complete vaccine nebulization and fill the mist storage tank according to parameters of nebulizer provided by vaccine manufactures and vaccine nebulization;
Step 5: forwarding the mist storage tank out, instructing the vaccine recipient to take the mist storage tank from the mist storage tank extraction outlet through the human-computer interaction module and guiding the vaccine recipient to complete vaccine inhalation;
Step 6: generating a vaccination record of vaccine nebulization and saving it on the cloud serve.

As an improvement, performing a step 5A after the step 5, wherein the step 5A including: controlling the inhalation quality detection module to determine whether a vaccination process is normally run according to preset vaccination quality standards; if not, generating notifications for manual intervention or activating preset operations.

As an improvement, performing a step 5B after the step 5A, wherein the step 5B including: controlling the disposable mist storage tank recovery device to open the mist storage tank recovery opening; guiding the vaccine recipient to put used mist storage tank to the mist storage tank recovery opening by the human-machine interface of the human-machine interaction module; starting medical waste procedures when it is detected that the mist storage tank is put in.

As an improvement, the step 1 further includes an automatic recognition and storage procedure including: S 1: obtaining a vaccine code; S2: retrieving vaccine storage parameters, nebulization parameters and usage parameters; S3: detecting whether vaccines are put in; S4: starting executing vaccine storage program, and automatically setting storage environment conditions according to the vaccine storage parameters, nebulization parameters and usage parameters.

As an improvement, in the step 1, conditions for the determination whether the vaccination is allowed include: whether the age falls within the permissible vaccination range, whether they belong to the category that should not receive a vaccine, whether they belong to the group who should defer vaccination and whether the vaccination history is matched with vaccines disposed in the intelligent vaccine nebulization system; if vaccination criteria are met, entering into the next step, otherwise exit.

### ADVANTAGEOUS EFFECTS OF THE PRESENT INVENTION

### Advantageous Effects

Compared with the prior art, the present invention has many advantages and positive effects: the invention relates to an intelligent vaccine nebulization system and a method of use and its advantages are to realize an automatic vaccination process of nebulization vaccine to minimize manual intervention, improve utilization rate and turnover rate of nebulizer; the vaccination process is standardized, the quality is controllable, and the entire vaccination process could be traced. Specific advantages and positive effects include:
1. The vaccination registration is automated. It can automatically determine whether the vaccine recipient meets vaccination requirements and which vaccine type should be administered based on the recipient's personal information.
2. Automated and standardized vaccine storage is achieved. The present invention can automatically provide a suitable vaccine storage environment based on parameters such as temperature, humidity, production date, and shelf life. It also regularly checks the validity period of vaccines and issues warnings when expired vaccines are detected.
3. Automatic and precise vaccine nebulization is achieved. The present invention can automatically and accurately extract specified doses of the vaccine for nebulization according to preset dose requirements.
4. An automated and efficient distribution of vaccine aerosol is realized. By introducing a brand-new mist storage tank, the utilization rate and turnover rate of the nebulizer are maximized. Additionally, it effectively prevents cross-infection caused by the vaccine nebulizer and saves costs associated with disinfection and maintenance.
5. Intelligent inhalation quality detection ensures the standardization of vaccine nebulization, maintaining control over the vaccination quality to guarantee the effectiveness of the vaccination.
6. The mist storage tank can be automatically recovered and disinfected, effectively avoiding secondary pollution during the vaccination process, thereby protecting the environment and reducing the workload for medical staff.
7. The entire process of nebulization vaccination can be automatically recorded to ensure traceability. Information is uploaded and stored in the cloud server, ensuring that the vaccine recipient can receive consistent, standardized, and high-quality vaccination on each intelligent vaccine nebulization system at any time.
8. The automation of the entire vaccination process, from registration to medical waste recycling, is realized. Manual intervention is minimized, leading to improved efficiency and vaccination quality, reduced workload for medical staff, and cost savings. Furthermore, it helps avoid mistakes caused by manual operation.

The present invention could improve the popularity of vaccination. The whole process of operation does not require manual intervention and could be deployed on any occasion.

### BRIEF DESCRIPTION OF THE DRAWINGS

### Description of the Drawings

Fig. 1 is a logical functional block diagram of an intelligent vaccine nebulization system according to one aspect of the present invention;
Fig. 2 is a flow chart of a method of using the intelligent vaccine nebulization system according to one aspect of the present invention;
Fig. 3 is a block diagram of a workflow of a vaccine information input module in the intelligent vaccine nebulization system according to one aspect of the present invention;
Fig. 4 is a block diagram of a workflow of a quality detection module in the intelligent vaccine nebulization system according to one aspect of the present invention;
Fig. 5 is a perspective view of a main cabinet in the intelligent vaccine nebulization system according to one aspect of the present invention;
Fig. 6 is a schematic diagram of a vaccine information input module in the intelligent vaccine nebulization system according to one aspect of the present invention;
Fig. 7 shows a working state of an inhalation quality detection module in the intelligent vaccine nebulization system according to one aspect of the present invention;
Fig. 8 is a perspective view of a mist storage tank used in the intelligent vaccine nebulization system according to one aspect of the present invention;
Fig. 9 shows a state of the intelligent vaccine nebulization system according to one aspect of the present invention in which the mist storage tank is being detected by a working process detection sensor device;
Fig. 10 is a schematic structural diagram of a vaccine quantitative removal module in the intelligent vaccine nebulization system according to one aspect of the present invention;
Fig. 11 is a schematic diagram of an automatic filling and taking structure for the mist storage tank in the intelligent vaccine nebulization system according to one aspect of the present invention.

In the figures, 1-temporary vaccine storage module, 1.1-vaccine temporary storage rack, 1.2-vaccine information scanner, 1.3-vaccine bottle, 1.4-pipette needle, 1.5-programmable dosing pump, 1.6-X rail, 1. 7 - Y rail, 1.8-Z rail, 2-communication interface, 3-mist storage tank management module, 3.1-mist storage tank management chamber door, 3.2-mist storage tank management door handle, 4-disposable mist storage tank recovery device, 4.1-mist storage tank recovery opening, 4.2-mist storage tank recovery chamber door handle, 4.3- mist storage tank recovery chamber door, 5-detection camera, 6-detection camera display, 7-display module, 8-audio guide module, 9-mist storage tank extraction outlet, 10-identity information input module, 11-mist storage tank, 11.1-one-way valve, 11.2-tank body, 11.3-cover, 12-working process detection sensor device, 13-horizontal driving device, 13.1-claw locking device, 13.2-automatic chuck device, 14. vertical lifting device.

### DETAILED DESCRIPTION OF THE DISCLOSURE

The present invention will be further described in detail below with reference to the accompanying drawings and embodiments.

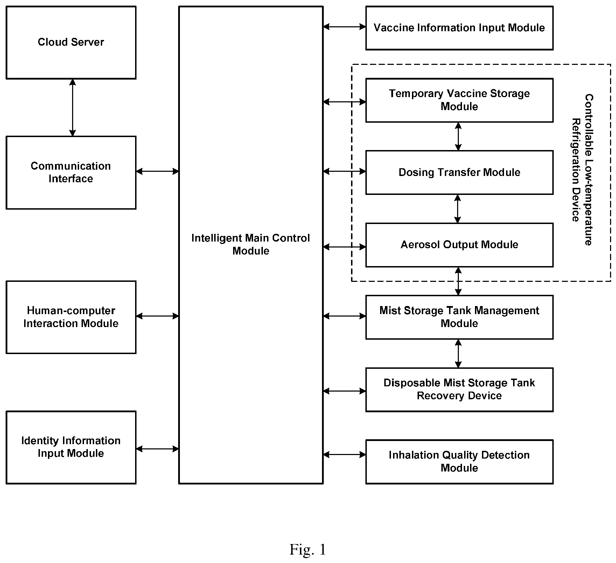

An intelligent vaccine nebulization system provided by the present invention is show in Fig. 1 to Fig. 10, which includes an intelligent vaccine nebulization device, a mist storage tank 11 and a cloud server. The intelligent vaccine nebulization device includes a main cabinet, an intelligent main control module and a plurality of functional modules provided on the main cabinet, wherein the functional modules include an identity information input module 10, a vaccine information input module, a temporary vaccine storage module, a dosing transfer module, an aerosol output module, a mist storage tank management module, a human-computer interaction module and a communication interface 2. The intelligent main control module is respectively connected with the functional modules and the intelligent main control module is communicated with the cloud server.

Each functional module is detailed as follows.

The identity information input module 10 is configured to input ID information of the vaccine recipient and send it to the cloud server through the intelligent main control module for verification, or recommend vaccine manufactures and vaccine types based on the recipient's age and health data.

The vaccine information input module is configured to identify vaccine manufacturers, vaccine types and production batch numbers, and supports user-defined or automatic downloads of storage parameters, nebulization parameters and usage parameters from the cloud server based on the vaccine types.

The temporary vaccine storage module 1 is configured to accommodate vaccine containers from different vaccine manufacturers, ensuring proper storage conditions to maintain the vaccine's effectiveness throughout the vaccination process.

The dosing transfer module is configured to automatically and accurately extract specified vaccine dosage according to different nebulized vaccination requirements and to transfer the measured amount of vaccine to the aerosol output module.

The aerosol output module is configured to automatically complete the nebulization based on the specific nebulization parameters for each vaccine, which is provided with an aerosol outlet through which the aerosol is filled into the mist storage tank.

The mist storage tank management module 3 is configured to perform detections and to install the mist storage tank 11 before nebulization, and is used to distribute the mist storage tank 11 , in which the nebulized vaccine is filled into, to the vaccine recipient for inhalation; the pre-nebulization installation is to connect the mist storage tank 11 to the aerosol outlet of the aerosol output module and the pre-nebulization detection is to check whether the mist storage tank 11 is inserted into the aerosol outlet and is in place.

The human-computer interaction module is configured to display vaccination information and a human-machine interface, providing prompts to the management personnel and the vaccine recipients.

The communication interface 2 is configured to connect to communicable devices, which can perform uplink communications or downlink communications.

The intelligent main control module is configured to control the functional modules of the intelligent vaccine nebulization system, to access the information of the vaccine recipient and other nebulization-related information on the cloud server, and to complete the control of a whole process including vaccine storage, nebulization and mist storage tank output based on the vaccine recipient and vaccine information.

The mist storage tank 11 serves as a container for storing vaccine aerosol, which is connected to the aerosol output module to receive the vaccine aerosol produced by the aerosol output module that is inhaled by the vaccine recipient for vaccination.

The cloud server is configured to interact with the intelligent main control module to access vaccination information, vaccine information and the vaccine recipient information, which is selectively networked with national or local disease control centers for data exchange.

The main cabinet is configured to accommodate the functional modules and used to receive and store the vaccines and the mist storage tank and to output the mist storage tank.

Furthermore, the intelligent vaccine nebulization system includes an inhalation quality detection module, which is configured to verify if a vaccination process is normally run according to predetermined vaccination quality standards. If not, the inhalation quality detection module automatically sends notifications for manual intervention or initiates preset operations.

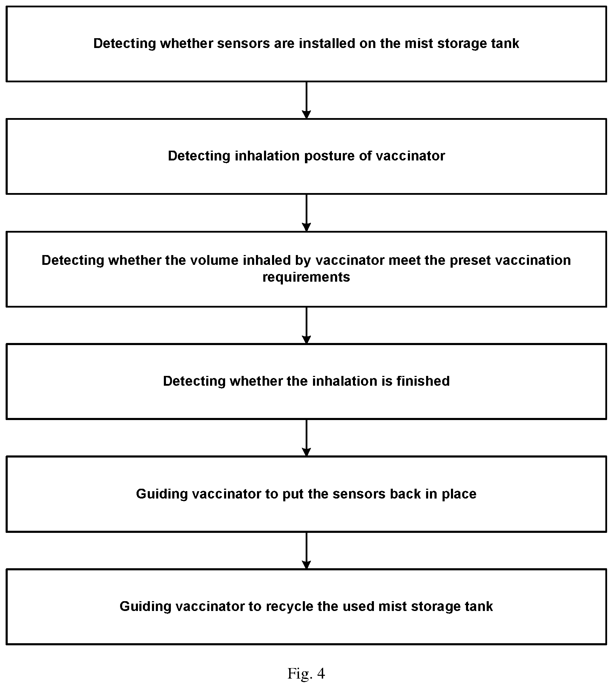

Furthermore, as shown in Fig. 4, Fig. 7 and Fig. 9, the inhalation quality detection module includes a detection camera device or/and a working process detection sensor device 12. The working process detection sensor device 12 includes an installation detection sensor, a photoelectric sensor, a body posture sensor and an air pressure sensor, which is configured to determine whether the inhalation posture and the volume inhaled by the vaccine recipient meet the preset vaccination requirements by providing feedbacks through the human-machine interface on the human-computer interaction module, and to determine whether a vaccination process is completed on the basis of inhalation detected by providing feedbacks on the human-machine interface on the human-computer interaction module; and then the vaccine recipient is instructed by the human-machine interface of the human-computer interaction module to put the working process detection sensor device 12 to its original position. The working process detection sensor device 12 is sleeved on the mist storage tank 11 in usage.

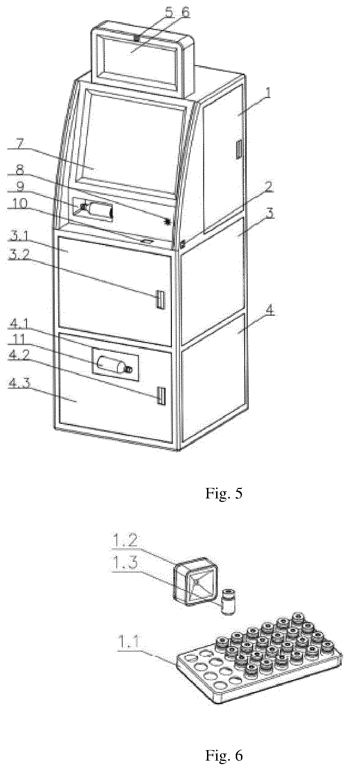

As shown in Fig. 5, the intelligent vaccine nebulization system further includes a disposable mist storage tank recovery device 4 configured to recover and treat medical wastes. The vaccine recipient is instructed by the human-machine interface of the human-computer interaction module to put the used mist storage tank 11 to designated recycling place.

To be specific, as shown in Fig. 5, the disposable mist storage tank recovery device 4 includes a waste recovery processing chamber, a mist storage tank recovery opening 4.1 and a mist storage tank recovery chamber door 4.3; wherein the mist storage tank recovery chamber door 4.3 may also be provided with a mist storage tank recovery chamber door handle 4.2. The mist storage tank management module 3 includes a mist storage tank management chamber and a mist storage tank management chamber door 3.1; wherein the mist storage tank management chamber door 3.1 may also be provided with a mist storage tank management chamber door handle 3.2.

As shown in Fig. 1 and Fig. 5, the aerosol output module includes a mist generation device, a mist storage tank output device and a mist storage tank extraction outlet 9.

As shown in Fig. 5 and Fig. 6, the vaccine temporary storage module 1 includes a vaccine storage chamber, a vaccine storage chamber door, a vaccine temporary storage rack 1.1 and a controllable low-temperature refrigeration device configured to maintain vaccines in a low-temperature environment. Further, the controllable low-temperature refrigeration device is also configured to maintain the temperature of the dosing transfer module and the aerosol output module.

As shown in Fig. 5, the main cabinet includes a shell. The communication interface 2, the human-computer interaction module, the mist storage tank management chamber door 3.1, the mist storage tank extraction outlet 9, the mist storage tank recovery opening 4.1, the vaccine storage chamber door and the identity information input module are disposed on the shell.

As shown in Fig. 5, the human-computer interaction module includes a display module 7 and an audio guide module 8; the display module 7 is configured to display vaccination information and the human-machine interface, and the audio guide module 8 is configured to generate guiding or warning sounds to management personnel or the vaccine recipient. The display module 7 and the audio guide module 8 are provided on a front part of the shell of the main cabinet. The detection camera device includes a detection camera 5 and a detection camera display 6, and the detection camera 5 and the detection camera display 6 are provided on a top part of the shell of the main cabinet.

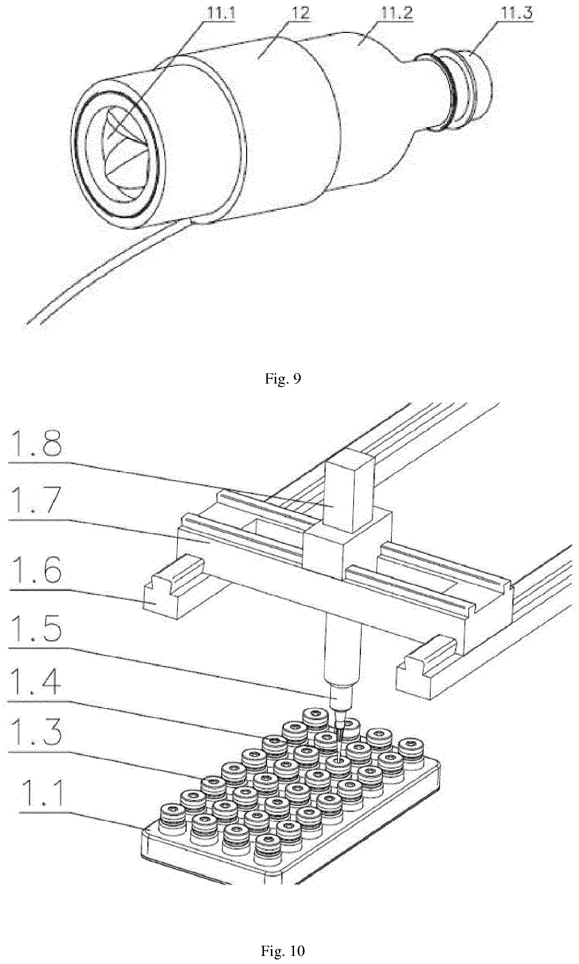

As shown in Fig. 6 and Fig. 10, the dosing transfer module includes a vaccine information scanner 1.2, a pipetting needle 1.4, a programmable dosing pump 1.5 and a three-dimensional moving device. The vaccine information scanner 1.2 is arranged in the vaccine storage chamber. The pipetting needle 1.4 is connected to the programmable dosing pump 1.5 and the programmable dosing pump 1.5 is provided on the three-dimensional moving device. Both of the programmable dosing pump 1.5 and the three-dimensional moving device are controlled by the intelligent main control module. The three-dimensional moving device is composed of an X rail 1.6, a Y rail 1.7 and a Z rail 1.8, which facilitates the movement of the pipetting needle 1.4 and the programmable dosing pump 1.5 in three-dimensional space to swiftly access the required vaccine bottle 1.3 for extracting the vaccine.

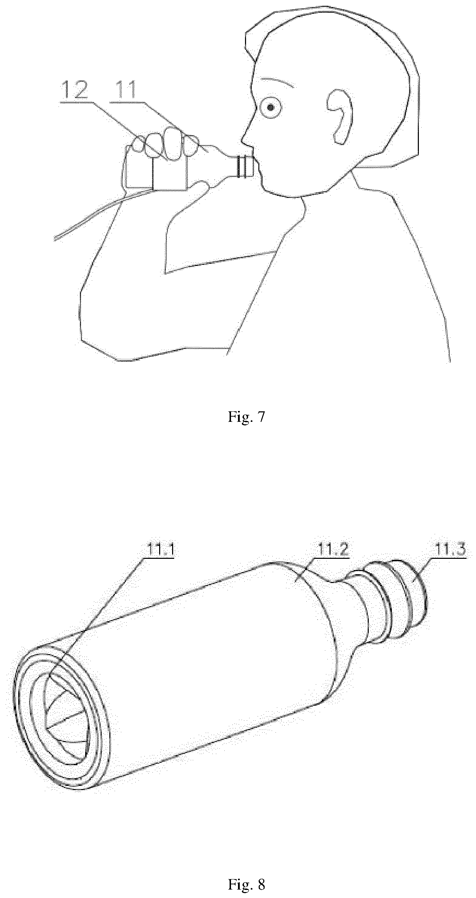

As shown in Fig. 8, the mist storage tank 11 includes a one-way valve 11.1, a tank body 11.2 and a cover 11.3; wherein the one-way valve 11.1 serves as the connection point between the mist storage tank 11 and the aerosol output module. When the mist storage tank 11 is disconnected from the aerosol output module, the one-way valve 11.1 could ensure the aerosol locked in the mist storage tank 11 and does not escape; when the user opens the cover 11.3 to inhale the aerosol from the mist storage tank 11, the air flows into the mist storage tank 11 through the one-way valve 11.1, which is convenient for the vaccine recipient to inhale the aerosol in the mist storage tank 11.

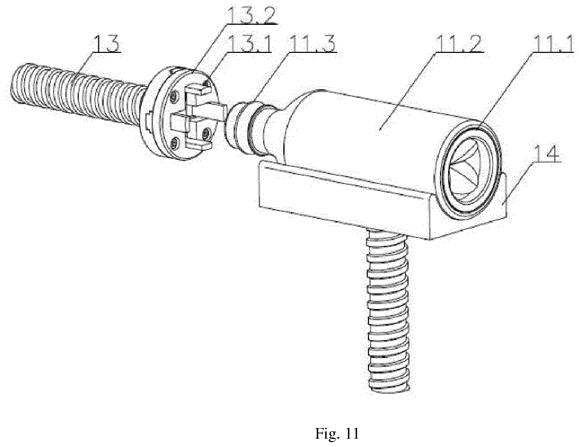

As shown in Fig. 11, an automatic loading and unloading mist storage tank structure provided in the mist storage tank management module 3 includes a horizontal driving device 13 and a vertical lifting device 14. The horizontal driving device 13 includes a claw locking device 13.1 and an automatic chuck device 13.2.

The vertical lifting device 14 lifts the mist storage tank 11 to the aerosol outlet of the aerosol output module, the one-way valve 11.1 of the mist storage tank 11 aligns with the aerosol outlet, the horizontal driving device 13 pushes the mist storage tank 11 to move laterally to insert it into the aerosol outlet to realize the automatic installation of the mist storage tank 11. After the aerosol is fully filled, the claws of the automatic chuck device 13.2 automatically grip the cover 11.3 of the mist storage tank 11; when the horizontal driving device 13 pulls the mist storage tank 11 to separate away from the aerosol output, the claws of the automatic chuck device 13.2 release the cover 11.3 of the mist storage tank 11 to realize an automatic separation of the mist storage tank 11 and the mist storage tank 11 falls into the mist storage tank extraction outlet 9.

A pressure sensor or a light sensor is installed at the aerosol outlet of the aerosol output module, which is used to determine whether the mist storage tank 11 is inserted into the aerosol outlet and in place. The mist storage tank management module 3 connects to the pressure sensor and the light sensor to perform the detection before nebulization.

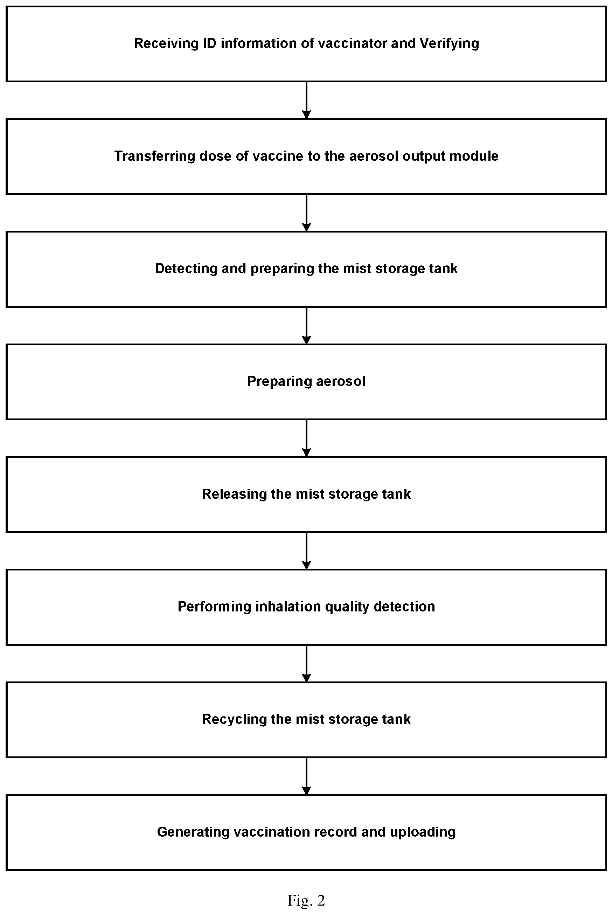

Referring to Fig. 2, a method of using the intelligent vaccine nebulization system including the following steps:
Step 1: Reading the vaccine recipient's ID information, connecting to the cloud server, retrieving personal information, vaccine type and vaccination requirements to determine whether the vaccination is permitted.
Step 2: Controlling the dosing transfer module to automatically and accurately extract required vaccine dosage according to dosage requirement of vaccine nebulization and depositing it into the aerosol output module.
Step 3: Controlling the mist storage tank management module to determine whether the mist storage tank 11 is installed in place; if usage requirements are not met, the mist storage tank is automatically installed or replaced.
Step 4: Controlling the aerosol output module to automatically complete vaccine nebulization and fill the mist storage tank 11 according to parameters of nebulizer provided by vaccine manufactures and vaccine nebulization.
Step 5: Forwarding the mist storage tank 11 out, instructing the vaccine recipient to take the mist storage tank 11 from the mist storage tank extraction outlet 9 through the human-computer interaction module and guiding the vaccine recipient to complete vaccine inhalation.
Step 6: Generating a vaccination record of vaccine nebulization and saving it on the cloud server.

Furthermore a step 5A is performed after the step 5. The step 5A includes: controlling the inhalation quality detection module to verify whether a vaccination process is normally run according to preset vaccination quality standards; if not, generating notifications for manual intervention or automatically triggers preset operations. The specific procedures are shown in Fig. 4.

Furthermore a step 5B is performed after the step 5A. The step 5B includes: controlling the disposable mist storage tank recovery device 4 to open the mist storage tank recovery opening 4.1; guiding the vaccine recipient to put used mist storage tank 11 to the mist storage tank recovery opening 4.1 by the human-machine interface of the human-machine interaction module; starting medical waste procedures, such as disinfection, crushing and the like upon detection of the placement of the mist storage tank 11.

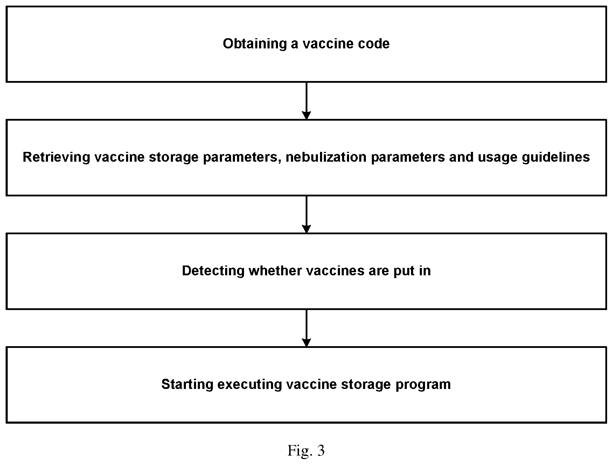

Furthermore, referring to Fig. 3, the step 1 also includes an automatic recognition and storage procedure including: S 1: obtaining a vaccine code; S2: retrieving vaccine storage parameters, nebulization parameters and usage parameters; S3: detecting whether vaccines are put in; S4: starting executing vaccine storage program, and automatically setting storage environment conditions according to the vaccine storage parameters, nebulization parameters and usage parameters. It could regularly check the validity period of vaccines and generate warnings for expired doses to take preset treatment measures.

Preferably, in the step 1, conditions for the determination whether the vaccination is allowed include: whether the age falls within the permissible vaccination range, whether they belong to the category that should not receive a vaccine, whether they belong to the group who should defer vaccination and whether the vaccination history is matched with vaccines disposed in the intelligent vaccine nebulization system. If the vaccination criteria are met, entering into the next step, otherwise exit.

The above are only the preferred embodiments of the present invention, and are not intended to limit the present invention in other forms. Any person familiar with the profession may use the technical content disclosed above to change or modify the equivalent of changes. However, any simple modifications, equivalent changes and modifications made to the above embodiments based on the technical essence of the present invention without departing from the content of the technical solution of the present invention still belong to the protection scope of the technical solution of the present invention.

## Claims

1. An intelligent vaccine nebulization system, **characterized by** comprising: an intelligent vaccine nebulization device, a mist storage tank and a cloud server, wherein the intelligent vaccine nebulization device includes a main cabinet, an intelligent main control module and a plurality of functional modules provided on the main cabinet, wherein the functional modules include an identity information input module, a vaccine information input module, a temporary vaccine storage module, a dosing transfer module, an aerosol output module, a mist storage tank management module, a human-computer interaction module and a communication interface, in which the intelligent main control module is respectively connected with the functional modules and the intelligent main control module is communicated with the cloud server;
the identity information input module is configured to input ID information of the vaccine recipient and send it the cloud server through the intelligent main control module;
the vaccine information input module is configured to identify vaccine manufacturers, vaccine types and production batch numbers and supports user-defined or automatic downloads of storage parameters, nebulization parameters and usage parameters from the cloud server based on the vaccine types;
the temporary vaccine storage module is configured to accommodate vaccine containers from different vaccine manufacturers, ensuring proper storage conditions to maintain the vaccine's effectiveness throughout the vaccination process;
the dosing transfer module is configured to automatically and accurately extract specified vaccine dosage according to different nebulized vaccination requirements and to transfer the measured amount of vaccine to the aerosol output module;
the aerosol output module is configured to automatically complete the nebulization based on the specific nebulization parameters for each vaccine, which is provided with an aerosol outlet through which the aerosol is filled into the mist storage tank;
the mist storage tank management module is configured to perform detection and to install the mist storage tank before nebulization, to distribute the mist storage tank, in which vaccine after nebulization by the aerosol output module is filled into, to the vaccine recipient for inhalation, in which the installation of the mist storage tank is to connect the mist storage tank to the aerosol outlet of the aerosol output module and the detection before nebulization is to check whether the mist storage tank is inserted into the aerosol outlet and is in place;
the human-computer interaction module is configured to display vaccination information and a human-machine interface, providing prompts to the management personnel and the vaccine recipients;
the communication interface is configured to connect to communicable devices;
the intelligent main control module is configured to control the functional modules of the intelligent vaccine nebulization system, to access the information of the vaccine recipient and other nebulization-related information on the cloud server, and to complete the control of a whole process including vaccine storage, nebulization and mist storage tank output based on the vaccine recipient and vaccine information;
the mist storage tank is a container for storing vaccine aerosol, which is connected to the aerosol output module to receive the vaccine aerosol produced by the aerosol output module that is inhaled by the vaccine recipient for vaccination;
the cloud server is configured to interact with the intelligent main control module to access vaccination information, vaccine information and the vaccine recipient information; and
the main cabinet is configured to accommodate the functional modules and used to receive and store the vaccines and the mist storage tank and to output the mist storage tank.

2. The intelligent vaccine nebulization system according to claim 1, wherein further includes an inhalation quality detection module configured to verify if a vaccination process is normally run according to predetermined vaccination quality standards; if not, to generate notifications for manual intervention or to initiate preset operations automatically.

3. The intelligent vaccine nebulization system according to claim 2, wherein the inhalation quality detection module includes a detection camera device or/and a working process detection sensor device; wherein the working process detection sensor device includes an installation detection sensor, a photoelectric sensor, a body posture sensor and an air pressure sensor, which is configured to determine whether the inhalation posture and the volume inhaled by the vaccine recipient meet the preset vaccination requirements by providing feedbacks through the human-machine interface on the human-computer interaction module, and to determine whether a vaccination process is finished on the basis of inhalation detected by providing feedbacks on the human-machine interface on the human-computer interaction module; and the vaccine recipient is instructed by the human-machine interface of the human-computer interaction module to put the working process detection sensor device back in place.

4. The intelligent vaccine nebulization system according to any one from claim 1 to 3, wherein further includes a disposable mist storage tank recovery device configured to recover and treat medical wastes, and the vaccine recipient is instructed by the human-machine interface of the human-computer interaction module to put the used mist storage tank to designated recycling place.

5. The intelligent vaccine nebulization system according to claim 4, wherein the disposable mist storage tank recovery device includes a waste recovery processing chamber, a mist storage tank recovery opening and a mist storage tank recovery chamber door; the mist storage tank management module includes a mist storage tank management chamber and a mist storage tank management chamber door; the aerosol output module includes a mist generation device, a mist storage tank output device and a mist storage tank extraction outlet; and the vaccine temporary storage module includes a vaccine storage chamber, a vaccine storage chamber door, a vaccine temporary storage rack and a controllable low-temperature refrigeration device configured to maintain vaccines in a low-temperature environment; the controllable low-temperature refrigeration device is further configured to maintain the temperature of the dosing transfer module and the aerosol output module.

6. The intelligent vaccine nebulization system according to claim 5, wherein the main cabinet includes a shell, wherein the communication interface, the human-computer interaction module, the mist storage tank management chamber door, the mist storage tank extraction outlet, the mist storage tank recovery opening, the vaccine storage chamber door and the identity information input module are disposed on the shell.

7. The intelligent vaccine nebulization system according to claim 3, wherein the human-computer interaction module includes a display module and an audio guide module; the display module is configured to display vaccination information and the human-machine interface, and the audio guide module is configured to generate guiding or warning sounds to management personnel or the vaccine recipient; the camera device includes a detection camera and a detection camera display and the detection camera and the detection camera display are provided on a top part of the shell of the main cabinet.

8. The intelligent vaccine nebulization system according to any one from claim 1 to 3, wherein the dosing transfer module includes a vaccine information scanner, a pipetting needle, a programmable dosing pump and a three-dimensional moving device, wherein the vaccine information scanner is arranged in the vaccine storage chamber, the pipetting needle is connected to the programmable dosing pump and the programmable dosing pump is provided on the three-dimensional moving device; the programmable dosing pump and the three-dimensional moving device are controlled by the intelligent main control module.

9. The intelligent vaccine nebulization system according to any one from claim 1 to 3, wherein the mist storage tank includes a one-way valve, a tank body and a cover; wherein the one-way valve serves as the connection point between the mist storage tank and the aerosol output module; when the mist storage tank is disconnected from the aerosol output module, the one-way valve ensures that the aerosol locked in the mist storage tank and does not escape; when the user opens the cover to inhale the aerosol in the mist storage tank, the air flows into the mist storage tank through the one-way valve, which is convenient for the vaccine recipient to inhale the aerosol in the mist storage tank.

10. A method of using the intelligent vaccine nebulization system according to any one from claim 1 to 9, wherein including:
Step 1: reading the vaccine recipient's ID information, connecting to the cloud server, retrieving personal information, vaccine type and vaccination requirements to determine whether the vaccination is permitted;
Step 2: controlling the dosing transfer module to automatically and accurately extract required vaccine dosage according to dosage requirement of vaccine nebulization and depositing it into the aerosol output module;
Step 3: controlling the mist storage tank management module to determine whether the mist storage tank is installed in place; if usage requirements are not met, the mist storage tank is automatically installed or replaced;
Step 4: controlling the aerosol output module to automatically complete vaccine nebulization and fill the mist storage tank according to parameters of nebulizer provided by vaccine manufactures and
vaccine nebulization;
Step 5: forwarding the mist storage tank out, instructing the vaccine recipient to take the mist storage tank from the mist storage tank extraction outlet through the human-computer interaction module and guiding the vaccine recipient to complete vaccine inhalation;
Step 6: generating a vaccination record of vaccine nebulization and saving it on the cloud server.

11. The method of using the intelligent vaccine nebulization system according to claim 10, wherein performing a step 5A after the step 5, wherein the step 5A including:
controlling the inhalation quality detection module to determine whether a vaccination process is normally run according to preset vaccination quality standards; if not, generating notifications for manual intervention or activating preset operations.

12. The method of using the intelligent vaccine nebulization system according to claim 11, wherein performing a step 5B after the step 5A, wherein the step 5B including: controlling the disposable mist storage tank recovery device to open the mist storage tank recovery opening; guiding the vaccine recipient to put used mist storage tank to the mist storage tank recovery opening by the human-machine interface of the human-machine interaction module; starting medical waste procedures when it is detected that the mist storage tank is put in.

13. The method of using the intelligent vaccine nebulization system according to claim 10 or claim 11, wherein the step 1 further includes an automatic recognition and storage procedure including: S1: obtaining a vaccine code; S2: retrieving vaccine storage parameters, nebulization parameters and usage parameters; S3: detecting whether vaccines are put in; S4: starting executing vaccine storage program, and automatically setting storage environment conditions according to the vaccine storage parameters, nebulization parameters and usage parameters.

14. The method of using the intelligent vaccine nebulization system according to claim 10 or claim 11, wherein in the step 1, conditions for the determination whether the vaccination is allowed include:
whether the age falls within the permissible vaccination range, whether they belong to the category that
should not receive a vaccine, whether they belong to the group who should defer vaccination and whether the vaccination history is matched with vaccines disposed in the intelligent vaccine nebulization system;
if vaccination criteria are met, entering into the next step, otherwise exit.
